## Europäisches Patentamt
## European Patent Office
## Office européen des brevets

(11) Veröffentlichungsnummer : **0 086 429**
**B1**

(12) **EUROPÄISCHE PATENTSCHRIFT**

(45) Veröffentlichungstag der Patentschrift :
09.08.89

(51) Int. Cl.⁴ : **A 61 B 5/04**

(21) Anmeldenummer : 83101139.0

(22) Anmeldetag : 07.02.83

(54) Verfahren zur Kardiogoniometrie und Kardiogoniometer dazu.

(30) Priorität : 12.02.82 CH 898/82

(43) Veröffentlichungstag der Anmeldung :
24.08.83 Patentblatt 83/34

(45) Bekanntmachung des Hinweises auf die Patenterteilung : 09.08.89 Patentblatt 89/32

(84) Benannte Vertragsstaaten :
AT BE CH DE FR GB IT LI LU NL SE

(73) Patentinhaber : **Sanz, Ernst, Dr. med.**
**CH-3532 Zäziwil (CH)**

(72) Erfinder : **Sanz, Ernst**
**CH-3532 Zäziwil (CH)**

(74) Vertreter : **Frei, Alexandra Sarah**
**Frei Patentanwaltsbüro Hedwigsteig 6 Postfach 95**
**CH-8029 Zürich (CH)**

(56) Entgegenhaltungen :
DE–A– 1 933 185
DE–A– 2 348 241
DE–A– 2 719 803
DE–B– 2 819 999
DE–B– 2 908 424
FR–A– 2 393 370
FR–A– 2 402 440
GB–A– 1 068 828
US–A– 4 249 538
J. PATZOLD: "Kompendium Elektromedizin; Grundlagen, Technik, Anwendungen", Siemens, 1976
BERLIN (DE)
INSTRUMENTENKUNDE, Jahrgang 57, April 1937.
H.E. und W. HOLLMANN: "Neue elektrokardiographische Registriermethoden", Seiten 147-167
PHILIPS TECHNISCHE RUNDSCHAU, Band 21, Nr. 2,
1959-1960. G.C.E. BURGER et al.: "Vektor-Elektrokardiographie", Seiten 51-65
COMPUTERS IN BIOLOGY AND MEDICINE, vol. 4, no.
2, Pergamon Press 1974 NEWCASTLE-UPON-TYNE
(GB). V. KALFF et al.: "A truly three-dimensional
vectorcardiographic display", pages 137-144

# EP 0 086 429 B1

## Beschreibung

Die Erfindung betrifft ein Verfahren zur Ermittlung und Darstellung eines auf das Herz bezogenen Raumvektors, der den Vektor des elektrischen Feldes darstellt, das durch die Aktivitaet des Herzens gebildet wird, ausgehend von Messwerten, die an Ableitungspunkten am Koerper erfasst werden, wobei aus den Messwerten an je zwei Ableitungspunkten je eine Potentialdifferenz gebildet wird.

Ein solches Verfahren ist zum Beispiel aus der Zeitschrift « Instrumentenkunde », Jahrgang 57, April 1937, H.E. und W. Hollmann : « Neue elektrokardiographische Registriermethoden », Seiten 147-167 bekannt. Diese Veroeffentlichung zeigt, dass drei Ableitungspunkte, und somit drei Elektroden, zur Erfassung von Messwerten am menschlichen Koerper so anzuordnen sind, dass diese Ableitungspunkte Ecken eines gleichseitigen Dreiecks bilden. Der geometrische Mittelpunkt dieses Dreiecks soll dabei mit dem Schwerpunkt des Herzens zusammenfallen. Zwischen je zwei Elektroden werden dann Spannungsdifferenzen gemessen, die man auch als Potentialdifferenzen bezeichnet. Diese Potentialdifferenzen werden als Projektionen eines Vektors auf die Seiten dieses Dreiecks aufgefasst. Ausgehend von diesen Projektionen werden Betrag und Richtung dieses Vektors in der Ebene dieses Dreiecks ermittelt. Dieser Vektor gibt Aufschluss ueber die Spannungen in einem elektrischen Feld, das durch das Herz erzeugt wird.

Der Nachteil dieses Verfahrens besteht darin, dass sich aus den genannten Potentialdifferenzen nur dann ein Vektor berechnen laesst, wenn die Potentiale an solchen Stellen gemessen werden, die zusammen Ecken eines gleichseitigen Dreiecks bilden. Verlaesst man diese Anordnung, so treten Fehler auf. Zudem wird auf diese Weise auch nur eine Projektion dieses Vektors auf eine Ebene ermittelt, was weitere Fehler zur Folge hat, denn der genannte Vektor liegt in Wirklichkeit nicht in dieser Ebene. Insbesondere laesst sich dieses Verfahren fuer rechtwinklige Ableitungssysteme nicht verwenden.

Aus dem Buch von J. Patzold : « Kompendium Elektromedizin ; Grundlagen, Technik, Anwendungen », Siemens, 1976 Berlin, Seiten 17 bis 20, sind zur Messung von elektrischen Spannungen am menschlichen Koerper weitere Ableitungspunkte bekannt. Diese von Nehb vorgeschlagenen Ableitungspunkte liegen in einer Ebene, die schraeg-sagittal das Herz schneidet. Die daraus abgeleiteten Potentialdifferenzen werden aber jede fuer sich als Skalare gedeutet und sind als Ergaenzung zu bekannten weiteren, aus anderen Punkten am Koerper abgeleiteten Potentialdifferenzen gedacht.

Der Nachteil dieses bekannten Verfahrens besteht darin, dass die einzelnen Potentialdifferenzen nur jede fuer sich betrachtet werden und allenfalls etwas ueber die Arbeit des Herzens aussagen sollen. Dagegen werden sie nicht zur Ermittlung eines Raumvektors herangezogen.

Ferner ist aus der Zeitschrift « Philips Technische Rundschau », Band 21, Nr. 2, 1959-1960, G. C. E. Burger et al : « Vektorelektrokardiographie », Seiten 51-65, bekannt, dass das Herz als Dipol aufgefasst und der Dipol als Vektor dargestellt werden kann. Unter der Voraussetzung, dass Potentiale an Elektroden gemessen werden, die so am Koerper eines Patienten angeordnet sind, dass sie Ecken eines rechtwinkligen Dreiecks bilden, wird ferner eine Beziehung zwischen gemessenen Potentialdifferenzen und dem Dipolvektor angegeben. Die gemessenen Potentialdifferenzen werden dabei als skalares Produkt aus der Projektion des Dipolvektors auf eine Seite des rechtwinkligen Dreiecks und aus einem weiteren Vektor betrachtet.

Der Nachteil dieses Verfahrens besteht aber darin, dass es schwierig ist, die Elektroden am Patienten so anzuordnen, dass die Berechnung des Vektors optimal erfolgen kann. Es ist somit immer noch mit groesseren Fehlern zu rechnen, insbesondere dann, wenn die Elektroden nicht an den Ecken des rechtwinkligen Dreieckes liegen. Zudem wird auf diese Weise auch nur die Projektion des Vektors auf eine Ebene ermittelt, in der der wirkliche Vektor auch nicht genau liegt.

Die Erfindung wie sie in den Patentanspruechen gekennzeichnet ist loest die Aufgabe, ein Verfahren zu schaffen mit denen, aus einer moeglichst geringen Anzahl Ableitungspunkte, Messwerte abgeleitet werden, die es erlauben, den Raumvektor mit seinem Betrag und seiner Lage im Raum moeglichst genau zu bestimmen.

Die durch die Erfindung erreichten Vorteile sind im wesentlichen darin zu sehen, dass aus Messwerten an nur vier Ableitungspunkten auf diese Art der Raumvektor genuegend genau ermittelt werden kann. Nachtraegliche Korrekturen wie sie aus den bisher verwendeten Verfahren bekannt sind, entfallen. Damit ist das erfindungsgemaesse Verfahren wesentlich einfacher durchzufuehren als die bisherigen Verfahren, bei denen etwa zwoelf Ableitungspunkte benoetigt wurden und die damit noch weniger genaue Resultate ergaben. Daraus ergibt sich auch ein erheblicher wirtschaftlicher Vorteil fuer die Anwender solcher Verfahren. Die Erfindung erlaubt es zudem erstmals orthogonale Ableitungen des Raumvektors der Herzens in einem System mit einer schraegsagittalen Ebene zu erzeugen und zwar ohne nachtraegliche Korrekturen zu benoetigen.

Die Erfindung wird nun mit Hilfe nachfolgend aufgeführter Figuren erklärt. Es zeigen :

Fig. 1 das Vierpunkte-Ableitungssystem gemäss der Erfindung,
Fig. 2 die Darstellung einer Projektion und Bildung eines Summenvektors daraus,
Fig. 3 die Bildung der Frontalebene mit drei Ableitungen,
Fig. 4 ein Quadrantenschema der schräg-sagittalen Ebene,
Fig. 5 einen Ausdruck von Herzparametern,

2

Fig. 6, 6' die Festlegung eines statistischen Normalbereichs für T- und R-Vektorrichtungen in kartesischer und polarer Koordinatendarstellung,

Fig. 7A, 7B in Prinzipdarstellung die Vorrichtung zur Durchführung des Verfahrens gemäss der Erfindung,

Fig. 8 einen Ausschnitt aus einer EKG-Kurve,

Fig. 9 ein Blockdiagramm einer Ausführungsform des Kardiogoniometers,

Fig. 10 eine Detaildarstellung aus Figur 9, und

Fig. 11 ein Struktogramm der Verfahrens.

Vor dem Eingehen auf die einzelnen Details soll noch in kommentierender Weise eine Uebersicht über einige wichtige Punkte gegeben werden.

Der Weg zu einer neuen Modellvorstellung des Dipolmomentes öffnet sich, wenn man sich von der Einzelableitung abwendet und die elektrischen Verhältnisse in einer durch drei Ableitungspunkte gebildeten Ebene als Ganzes in Betracht zieht. Verwendet man im EKG den Begriff der geschlossenen Masche, beispielsweise in Form der Nehb'schen Ableitungen D, A, J, und betrachtet die elektrischen Verhältnisse in diesem Dreieck, so kann aus der Grösse der Ausschläge des elektrokardiographen näherungsweise auf ein relatives Mass für die Grösse des Dipolmoments geschlossen werden. Natürlich werden die Simultanausschläge, ob gross oder klein, immer die Summe Null ergeben, entsprechend der oben angedeuteten kirchhoff'schen Maschenregel. Betrachtet man nun aber die durch die drei Ableitungen gegebenen Potentialdifferenzen als Teilvektoren und addiert sie alle drei nach den Regeln der vektoriellen Addition, indem man die Grösse der Auschläge und ihre Polarität einerseits, aber auch die Ableitungsrichtung andererseits berücksichtigt, so erhält man einen Summenvektor, den man im hier vorgestellten Modell als Dipolmoment des Herzens in seiner Gesamtheit bezeichnen kann,

$$\text{Vektor } \vec{V} = \vec{D} + \vec{A} + \vec{I}$$

Aufgrund dieser neuen Konzeption ist es möglich, von vier herznahen Ableitungspunkten aus und ohne Vorschaltung eines wie bis anhin notwendigen Widerstandsnetzwerkes drei orthogonale Projektionen x, y und z zu konstruieren.

Auch dieses neue Modell der Vektorkonstruktion kann naturgemäss nicht den Anspruch auf absolute Genauigkeit erheben. Dies beruht auf der inhomogenen Leitfähigkeit des das Herz umgebenden Gewebes, das für den einzelnen Patienten jedoch eine konstante Grösse darstellt, so dass man auf deren Korrektur verzichten kann, wie dies auch bei allen empirischen, sogenannten kubischen Systemen (Schellong, Duchosal, usw.) der Fall ist. Infolge der Inhomogenität des elektrischen Feldes und damit der Unkenntnis des Feldlinienverlaufes an den Ableitungspunkten gemäss Erfindung ist die Bestimmung des Dipols, sowohl was die Magnitude als auch die Richtung anbetrifft, wohl nicht ganz genau, aber für den einzelnen Patienten konstant und über Jahre reproduzierbar (was anhand von hunderten von Messungen ermittelt worden ist). Dieses Resultat ergab sich nicht zuletzt durch die einfache und anwendungssichere Ableitungstechnik gemäss der Erfindung.

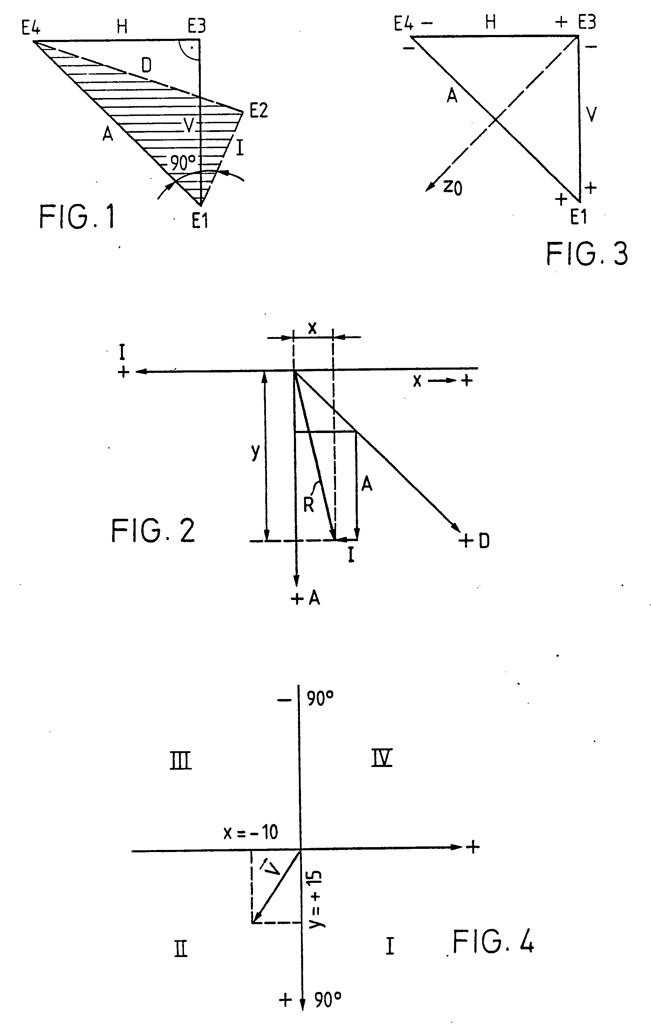

Figur 1 zeigt das Vier-Punkte-Ableitungssystem gemäss Erfindung. Das im räumlichen Sinne zu verstehende, tetraederförmige Ableitungssystem zeigt die vier Elektrodenansatzstellen E1, E2, E3 und E4. Verbunden sind diese Ableitungspunkte jeweils folgendermassen : (E1, E2) als inferiores Paar I, wobei E1 dem Punkt zur Ermittlung der Spannung V4 nach Wilson entspricht, und zwar 5 ICR (Intercostalraum) und MCL (Medioclavicularlinie), und der zu E1 sagittal liegende Punkt E2 dem Punkt zur Ermittlung der Spannung V8 nach Wilson entspricht. Lotrecht über dem Punkt E1 ist im Abstand von $1/\sqrt{2}$ mal $\overline{E1\ E2}$ der Ableitungspunkt E3 ; diese Ableitung ist die vertikale, mit V bezeichnete. Das horizontale, mit H bezeichnete Ableitungspaar (E3, E4) ist waagerecht nach der rechten Patientenseite im Abstand von $1/\sqrt{2}$ mal $\overline{E1 : E2}$ angeordnet. Dann wäre noch die dorsale, mit D bezeichnete Ableitung (E2, E4) sowie die anteriore, mit A bezeichnete Ableitung (E1, E4) zu nennen, deren Abstand zueinander durch die vorgenannten proportionalitäten festgelegt ist. Ein zusätzliches Lot vom Ableitungspunkt E3 auf die Anteriore wird mit $z_0$ bezeichnet. In dieser Darstellung bildet dann A/I die schräg-sagitale x, y-Ebene, Z/A die frontale y, z-Ebene und Z/I die z, x-Ebene. Die Paarung der Ableitungspunkte E2/ und E3 wird als einer unbenützten Ebene zugehörig sowie auch als redundantes Paar nicht berücksichtigt. Das heisst jedoch nicht, dass nicht auch aus dieser Ableitung diagnostisch relevante Information zu entnehmen ist.

Zwei Ableitungspunkte, nämlich E3 und E2 sind in ihrer relativen Lage zum Herzen des Patienten verhältnismässig unkritisch, d. h. Manipulationsstreuungen innerhalb gewisser Grenzen beim Anbringen der Elektroden führen jeweils zum selben Endresultat. Lotrecht über der Herzspitzenelektrode E1 — deren Lage übrigens recht genau bestimmt werden muss — kommt auf verhältnismässig verschieblichem Gewebe der Ableitungspunkt mit der Elektrode E3 zu liegen. Vor allen Dingen bei Lageänderungen des Patienten während der Messung wird dieser Ableitungspunkt E3 relativ zur angelegten Geometrie und damit auch zum Herzen in eine jeweils andere Lage verschoben. Dies führt jedoch nicht zu Fehlmessungen, so dass die am meisten den Lageverschiebungen ausgesetzte Elektrode E3 als eine elektrisch adaptive Elektrode betrachtet werden kann. Sie ist gegen Verschiebung des Gewebes beziehungsweise Lokalisierungsfehler wenig empfindlich, was als Vorteil der Messung angesehen werden kann. Die Messung wird bei Lageänderung des Patienten nicht wesentlich beeinflusst. Zur begrifflichen Präzisierung für das Nachfolgende soll noch erwähnt werden, dass zwischen x, y, z-Projektionen und den

3

entsprechenden elektrischen Ableitungen beziehungsweise deren Signalen jeweils unterschieden wird.

Die Elektroden werden für die Ableitung gemäss der Erfindung folgendermassen am Patienten angelegt :

1. E1 entsprechend dem Punkt für die Spannung V4 (Wilson) = 5 ICR und MCL ;
2. E2 sagittal zu E1 (entsprechend dem Punkt für die Spannung V8 Wilson) ;
3. E3 lotrecht über E1 im Abstand $0.7 \cdot \overline{E1 \quad E2}$ ;
4. E4 waagerecht nach der rechten Patientenseite im Abstand von $0.7 \cdot \overline{E1 \quad E2}$ ;
5. die Masseelektrode wird vorzugsweise am rechten Arm des Patienten angebracht.

Dies ist ein bevorzugtes Vorgehen zum Anlegen der Elektroden am Patienten. Dabei sei noch speziell darauf hingewiesen, dass für die Herzdiagnose an Tieren nur die für das bestimmte Tier geltende geometrische Elektrodenkonfiguration zur Ableitung angewendet und zur Bestimmung der orthogonalen Ableitungen lediglich die Koordinatentransformationsstufe mit adäquaten Parametern versehen werden muss. Die Erfindung eignet sich also gleichermassen für die Herzdiagnose vom Mensch und Tier.

Die Vektorschleife beim Herzgesunden verläuft von vorne oben rechts nach vorne unten links und nach Wendung gegen hinten wieder zurück nach vorne oben rechts, d. h. die Vektorschleife liegt mehr oder weniger in oder parallel einer zur Sagittalen geneigten Ebene (gemäss Figur 1 die Fläche zwischen den drei Ableitungspunkten E1, E2 und E4). EKG-Ableitungen in dieser Ebene müssen somit auf Veränderungen der Vektorschleife sehr sensibel reagieren, was an weit über 2000 EKG-Messungen bestätigt wurde. Diese Ebene wird zur Konstruktion zweier senkrecht aufeinanderstehender Projektionen x und y und die Frontalebene zur Konstruktion der senkrecht auf der Nehb'schen Ebene stehenden Projektion $z_0$ benutzt.

Damit ein rechtwinkliges Ableitungsdreieck erhalten wird, was aus trigonometrischen Gründen Vorteile bietet, wählt man als dorsalen Ableitungspunkt des zur Sagittalen geneigten Dreiecks nicht den Punkt V7, wie Nehb es tat, sondern E2 entsprechend V8, der schräg-sagittal zu Punkt V4 entsprechend E1 (= Herzspitze) liegt. Damit erhält man über der Herzspitze einen rechten Winkel, die Ableitung A steht senkrecht zur Ableitung I gemäss der Üblichen EKG-Terminologie ; zudem ist dieser Ableitungspunkt V8 entsprechend E2 eindeutig bestimmbar und leicht zu finden, ein Teilaspekt des Ableitungssystems gemäss Erfingung. Mit der Wahl des Punktes V8 nach Wilson als dorsalen Ableitungspunkt erhalten wir mit den Punkten $E_1$-$E_2$-$E_4$ eine Ebene definiert, die im Folgenden schräg-sagitalle ebene genannt wird. Die von Nehb eingeführten Ableitungsbezeichnungen D, A und I werden beibehalten.

Wählt man die y-Achse des hier besprochenen orthogonalen Systems parallel zur Ableitung A und die x-Achse parallel zur Ableitung I, so darf die Ableitung A nicht als Projektion des Vektors auf die Achse y und die Ableitung J nicht als Projektion des Vektors auf die Achse x angenommen werden. Es müssen vielmehr zur Vektorkonstruktion alle drei Ableitungen D, A und I vektoriell addiert werden in der Form V = D + A + I Den so gebildeten Summationsvektor projiziert man dann auf die x- und y-Achse, wie dies in Figur 2 dargestellt ist. Gemäss trigonometrischer berechnung erhält man folgende Formeln :

$$x = D \cos 45° - I = 0,7 \, D - I \qquad (1)$$

$$y = D \sin 45° + A = 0,7 \, D + A \qquad (2)$$

Als dritte Achse im orthogonalen System braucht man eine zusätzliche Vektorprojektion, die senkrecht auf diesen zwei Achsen steht. Diese Projektion $z_0$ erhält man durch die ableitung im frontalen Dreieck der Punkte E1, E3, E4, wobei die Punkte E1 und E4 die vorderen Ableitungspunkte des schräg-sagittalen Dreiecks darstellen. Punkt E3 wird so gewählt, dass seine Distanz von den Punkten E1 und E4 gleich gross ist und zwischen diesen zwei Linien ein rechter Winkel besteht, wie die in Figur 3 dargestellt ist. Damit erhält man ein rechtwinkliges, gleichschenkliges Dreieck. In diesem Dreieck gelten folgende Ableitungen : von Punkt E4 zu Punkt E3 die horizontale Ableitung H, von Punkt E3 zu Punkt E1 die vertikale Ableitung V und von Punkt E1 zu Punkt E4 die anteriore Ableitung A, welche mit der Ableitung A im schräg-sagittalen Dreieck D, A, I identisch ist. Auch in diesem frontalen Dreieck kann man nach dem gleichen Vorgang einen Vektor konstruieren, indem man die simultanausschläge der drei Ableitungen vorzeichen- und achsengerecht zur vektorsummation aneinanderreiht. Was von diesem Summenvektor nun interessiert, ist seine Projektion auf die in der figur 3 angegebene $z_0$-Achse. Diese $z_0$-Achse ist nichts anderes als die Senkrechte vom Punkt E3 auf die Ableitung A und damit auf die Nehb'sche Ebene. Da diese Senkrechte die Winkelhalbierende des rechten Winkels in Punkt E3 darstellt, lässt sich diese Projektion $z_0$ des Vektors trigonometrisch darstellen als $(V - H) \cdot \sin 45°$, wobei bei positivem Vorzeichen dieser Grösse der Vektor unter der Nehb'schen Ebene liegt, bei negativem Vorzeichen über der Nehb'schen Ebene. Anders ausgedrückt: die $z_0$-Achse ist somit nach unten gerichtet positiv, nach oben gerichtet negativ und stets senkrecht auf der schrägsagittalen Ebene, d. h. senkrecht zu den Achsen x und y. Zusammenfassend kann gesagt werden, die drei orthogonalen Projektionen x, y und z lauten somit :

$$x = D \cos 45° - I = 0,7 \, D - I \qquad (1)$$

$$y = D \sin 45° + A = 0{,}7\,D + A \qquad (2)$$

$$z = (V - H) \sin 45° = 0{,}7\,(V - H) \qquad (3)$$

Die Berechnung der Maximalvektoren von QRS und T und die Bestimmung des Raumwinkels $\varphi$ zwischen diesen beiden Maximalvektoren wird gemäss dem üblichen raumtrigonometrischen Vorgehen durchgeführt :

Die Raumvektoren seien bezeichnet mit 1 und 2, deren Projektionen auf die x-, y- und z-Achse mit $x_1$, $y_1$, $z_1$ bzw. mit $x_2$, $y_2$ und $z_2$, welche Grössen einem orthogonalen, räumlichen Koordinatensystem angehören.

Die zur Vektorberechnung gebräuchliche Formel für den Raumwinkel $\varphi$ lautet :

$$\cos \varphi = \frac{x_1\,x_2 + y_1\,y_2 + z_1\,z_2}{\sqrt{(x_1^2 + y_1^2 + z_1^2)(x_2^2 + y_2^2 + z_2^2)}} \qquad (4)$$

Im Zähler erhält man das Skalarprodukt und im Nenner das Produkt der Absolutwerte der beiden Vektoren. Damit beträgt die Länge des Vektors 1 :

$$\sqrt{(x_1^2 + y_1^2 + z_1^2)} \qquad (5')$$

die Länge des Vektors 2 beträgt :

$$\sqrt{(x_2^2 + y_2^2 + z_2^2)} \qquad (5'')$$

Der Maximalvektor der Depolarisation ist somit derjenige Punkt der Vektorschleife von QRS, dessen Summe der Quadrate der drei Projektionen x, y und z am grössten ist, also :

$$V_{max}(QRS) = \sqrt{(x_1^2 + y_1^2 + z_1^2)}^{\,max} \qquad (6')$$

Das selbe gilt für den Maximalvektor der T-Schleife :

$$V_{max}(T) = \sqrt{(x_2^2 + y_2^2 + z_2^2)}^{\,max} \qquad (6'')$$

Neben dem Winkel $\varphi$ interessiert auch die Lage der Maximalvektoren von QRS und T im Raume, weshalb die Projektionen der Vektoren in die schräg-sagittale Ebene einerseits und in die Frontalebene andererseits bestimmt werden müssen. Dabei ist zu bemerken, dass aus historischen Gründen in der Elektrokardiographie, insbesondere in der Vektorkardiographie, die Raumkoordinatenachsen in mathematisch nicht üblichem Sinne bezeichnet sind ; die x-, y- und z-Achsen sind relativ dem menschlichen Körper zugeordnet, wobei der Koordinatenursprung ungefähr zentral im Torso zu liegen kommt. Die in der Körperlänge nach unter und oben verlaufende Achse ist die y-Achse ; die negative y-Achse zeigt nach oben gegen den Kopf und die positive y-Achse zeigt nach unten. Die horizontale Achse ist die x-Achse, wobei nach rechts sich die positive x-Achse erstreckt, umgekehrt nach links die negative x-Achse. Quer durch den Körper von vorne nach hinten verläuft die z-Achse, und zwar von der Körpermitte nach vorne die positive z-Achse und nach hinten aus dem Rücken austretend die negative z-Achse. Dies entspricht der üblichen Konvention ; das für die Erfindung verwendete orthogonale System entspricht dem in Figur 1 gezeigten x, y, $z_o$-System, es ist um 45° zur Sagittalen geneigt.

Die schräg-sagittale Ebene — durch die Achsen x und y bestimmt — wird in 360° aufgeteilt, wobei 0° horizontal nach hinten, 90° nach unten links, 180° nach vorne und minus 90° nach oben rechts festgelegt werden ; dieser Sachverhalt ist in Figur 4 dargestellt. Die Umwandlung der rechtwinkligen Koordinaten x und y in die Polarkoordinaten erfolgt nach folgenden Formeln, wobei der Polarwinkel in der schräg-sagittalen Ebene mit $\alpha$ bezeichnet ist.

$$\text{Quadrant I : x positiv, y positiv} \quad\sphericalangle\; \alpha = \arctg \frac{+y}{+x} \qquad (7)$$

$$\text{Quadrant II : x negativ, y positiv} \quad\sphericalangle\; \alpha = \arctg \frac{+y}{-x} + 180° \qquad (8)$$

$$\text{Quadrant III : x negativ, y negativ} \quad\sphericalangle\; \alpha = \arctg \frac{-y}{-x} - 180° \qquad (9)$$

$$\text{Quadrant IV : x positiv, y negativ} \measuredangle \, \alpha = \text{arctg } \frac{-y}{+x} \qquad\qquad (10)$$

Ein Beispiel : x betrage — 10, y + 15. Dieser Vektor muss im II. Quadranten liegen :

$$\measuredangle \alpha = \text{arctg } \frac{15}{-10} + 180° = 124°$$

Dieses Beispiel ist im Quadrantenschema eingetragen (Fig. 4).

Genau gleich verfährt man mit der Umwandlung der rechtwinkligen Koordinaten z und y in der Frontalebene, indem man die y-Achse der schräg-sagittalen Ebene als Basis wählt. Null Grad ist somit links unten, 180° rechts schräg oben. Die Basis in der Frontalebene liegt somit 45° zur Körperachse, also schräg-sagittal, und nicht horizontal wie bei den Frank'schen Ableitungen. Der Polarwinkel in der Frontalebene wird mit β bezeichnet. Dieser Winkel β gibt die genaue Projektion des Vektors auf die Frontalebene ; ist β positiv, verläuft der Vektor unter der schräg-sagittalen Ebene, ist er negativ, über dieser.

Die Ermittlung der Maximalvektoren von QRS und von T aufgrund der Ableitungen D, A, I und H, A und V, d. h die Verwertung der elektrischen Ableitungssignale, ist Aufgabe des Kardiogoniometers gemäss der Erfindung. Von der vier Messpunkten E1, E2, E3, E4 (Figur 1) werden die fünf Ableitungssignale D (Dorsal), A (Anterior), I (Inferior), H (Horizontal) und V (Vertikal) gebildet und gemäss der oben genannten Formeln 1, 2 und 3 zu den drei Projektionen x, y und z gewandt. Die drei daraus entstehenden, zeitabhängigen, elektrischen Signale bilden drei, den Projektionen entsprechende Kurvenzüge, die in Zeitabständen von beispielsweise drei Millisekunden ausgemessen, quadriert und summiert werden und schliesslich diese Werte in einem Speicher zur Weiterverwertung gespeichert werden ; dies bezieht sich in diesem Beispiel dann einerseits auf die Werte QRS und andererseits auf die Werte T. Nach dem Wert T wird zwischen der T-Welle und der folgenden P-Welle des nächsten Schlages die Null-Linie ermittelt und entsprechende Korrekturen vorgenommen, wenn nötig alle die gespeicherten Werte auf den ermittelten Null-Wert bezogen. Das Maximale Summenquadrat von $x_1$, $y_1$ und $z_1$ einerseits und von $x_2$, $y_2$ und $z_2$ andererseits wird ermittelt. Aufgrund dieser Daten errechnet das Kardiogoniometer dann den cos φ nach Formel 4 und bestimmt daraus den arccos φ. Es wird also beinahe unmittelbar der Winkel φ eines bestimmten einzigen Herzschlages bereitgestellt. Anschliessend werden im Kardiogoniometer auch die Winkel αR, αT, βR und βT aufgrund der Formeln 7 bis 10 errechnet und diese Daten inklusive der Basisdaten $x_1$, $y_1$, $z_1$, $x_2$, $y_2$, und $z_2$ abgespeichert. Von ein und demselben Herzschlag sind damit elf Parameter bereitstellbar und als zwöfter Parameter steht noch das Schlagintervall zur Verfügung. Alternativ kann das Problem der langsam schwankenden Null-Linie auch mit einem digitalen oder analogen Hochpassfilter gelöst werden.

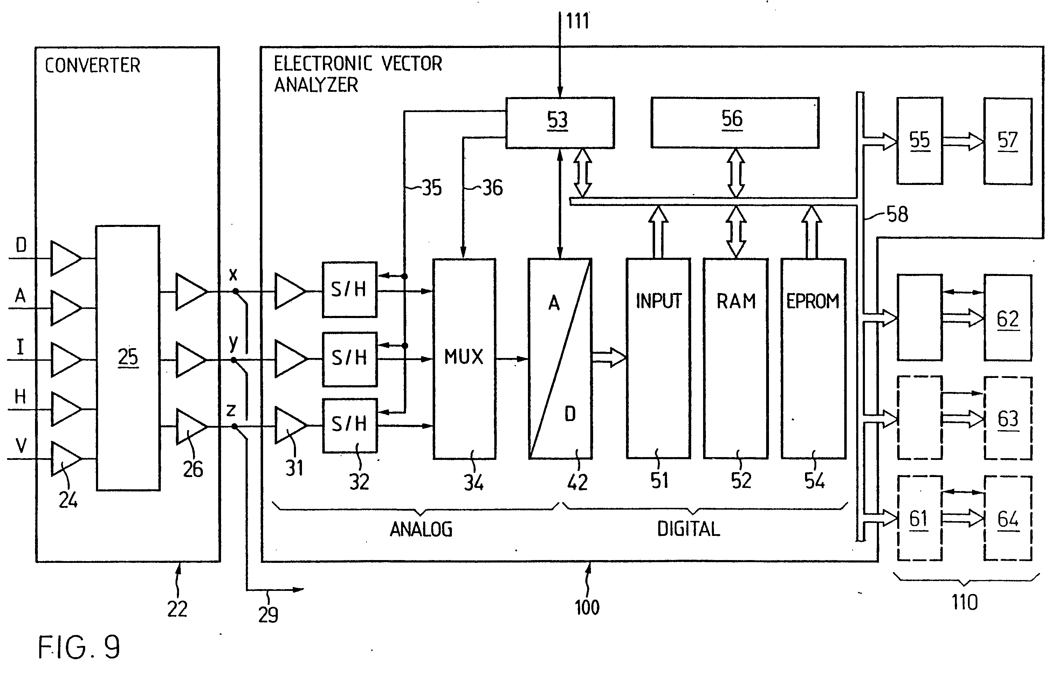

Zur Kontrolle des Kardiogoniometers im Betrieb können beispeilsweise gleichzeitig die Projektionen x, y und z auf einem Drei-Kanal-Elektrokardiographen mitgeschrieben werden (Figur 9). Diejenigen Signalkomplexe, die der Kardiogoniometer errechnet hat, werden auf dem die Kurvenzüge enthaltenden Streifen markiert. Damit steht einerseits eine Anzeige zur Verfügung, die sich an die übliche Darstellung des EKG anlehnt und andererseits zugleich eine graphische Darstellung einiger errechneter Daten bzw. wirklicher Projektionen der Ableitungen enthält.

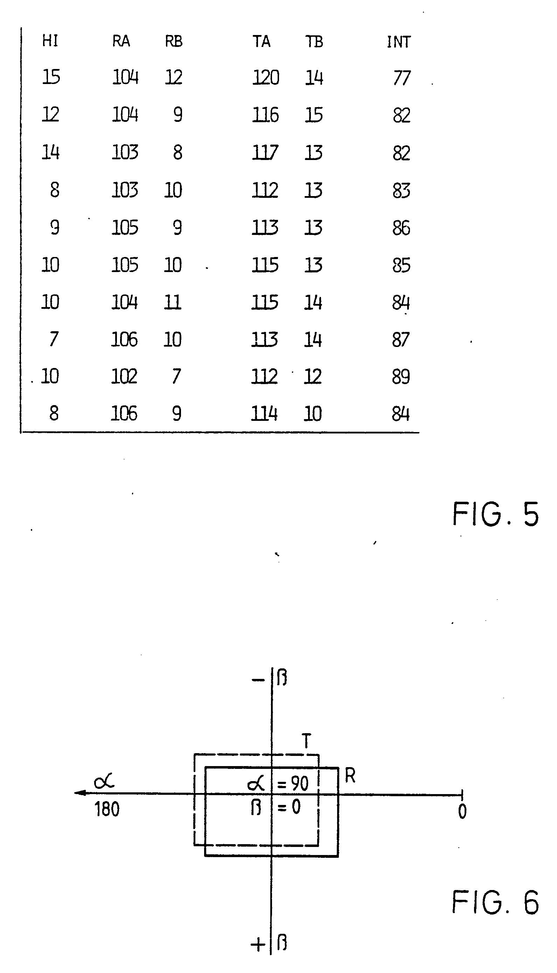

In einer verwendeten Ausführungsform des Kardiogoniometers werden sechs Messgrössen Phi, αR, βR, αT, βt sowie das Schlagintervall auf einem Drucker fortlaufend ausgedruckt (Figur 5). Auf diese Weise wird zirka jeder dritte Herzschlag ausgemessen. Es können also in kurzer zeit am Patienten Serienmessungen durchgeführt werden, die es erlauben, statistische Werte wie Mittelwert und Standardabweichung zu bestimmen.

Als Beispiel wurden die Normwerte an einem Kollektiv von hundert herzgesunden Probanden ermittelt, welche folgende Kriterien erfüllen :

1. Klinish keine Anhaltspunkte für eine organisches Herzleiden.
2. Normales EKG in den zwölf Standardableitungen gemäss Stand der Technik.
3. Ueber zehn Herzschläge konstante Werte aller fünf Parameter φ, αR, αT, βR und βT gemäss der Erfindung, wobei die Streuung kleiner als ± 5° ist, was bedeutet, dass bei jedem Herzschlage De- und Repolarisation den gleichen « elektrischen Weg » nehmen. Die so ermittelten Werte lauten für

| Phi | αR | βR | αT | βT |
|-----|-----|-----|-----|-----|
| $\bar{x}$ 15° | 89,9° | 9,4° | 98° | 3,2° |
| s 7,9° | 11,6° | 7,8° | 10,5° | 8,8° |

Wählt man ± 2 Standardabweichungen als Normgrenzen, ergeben sich folgende Normwerte (aufgerundet) für Herzgesunde in Rückenlage :

Phi = 0° bis 31°
αR = 65° bis 115°
βR = + 25°bis — 10°
αT = 75° bis 120°
βT = + 20° bis — 15°

In diesem Kollektiv finden sich 56 Männer und 44 Frauen ; das Alter der Probanden lag zwischen 14 und 89 Jahren. Mit plus/minus zwei Standardabweichungen sind 95,4 % der Fälle statistisch erfasst.

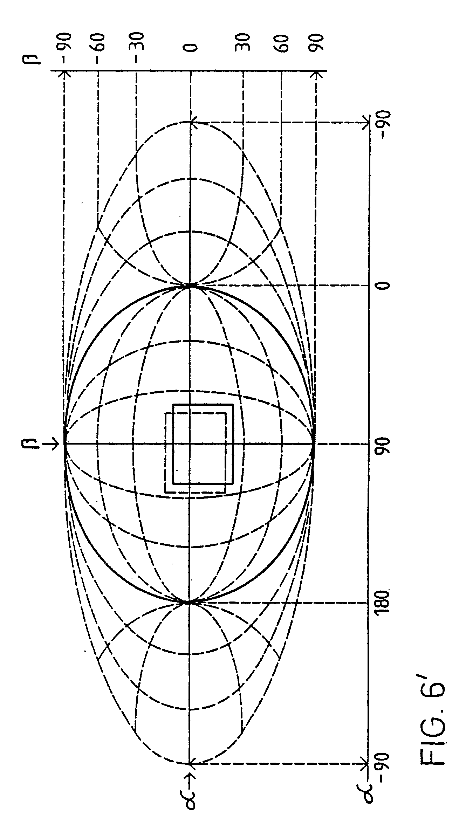

Die Maximalvektoren von De- und Repolarisation liegen beim Normalen somit sehr nahe beieinander, der wahre Winkel im Raum Phi beträgt 0° bis 31° (15° +/— 16°). Bei einem Winkel kleiner als 31° können aber trotzdem pathologische Verhältnisse vorliegen, wenn sowohl die De- wie auch Repolarisation gestört sind. Als Sehr wichtig erweist sich somit die Ortung von $R_{max}$ und $T_{max}$ in Bezug auf die Saggital- und Frontalebene. Diese liegen in einem kleinen Umkreis um die mittlere Axillarlinie (Winkel α = 90°) und wenig unter oder über der schrägsagittalen Ebene (Winkel β 0°). Figur 6 zeigt in einer grafischen Darstellung diesen eben beschriebenen Sachverhalt.

Ein Abweichen der Vektoren von diesem elektrischen Zentrum (gemäss Figur 6′ durch einen längeren Vektorpfeil angedeutet) nach vorne, hinten, oben und unten und schliesslich auch noch auf die Hinterfläche (damit ist die Darstellung auf der Kugeloberfläche gemäss Figur 6′ gemeint) bedeutet einen pathologischen Befund. So finden sich typische Verlagerungen des R-Vektors, z. B. bei den Blockbildern, LSB bzw. RSB, LAFB oder BFB sowie auch bei R-Verlusten nach Infarkten. Andererseits wird jede Repolarisationsstörung sich im Abweichen des T-Vektors in der Gegenrichtung zum Herd der Läsion manifestieren.

In Linksseitenlage verlagert sich das Herz meist um ungefähr 10° nach hinten. Die Normwerte verschieben sich also was den Winkel α betrifft um 10° nach hinten auf :

αR = 55° bis 105° ; αT = 65° bis 110°

Die bisher verbreitete Vorstellung, dass in Linkslage Durchblutungsstörungen der rechten Koronararterie (RCA) sich manifestieren könnten, hat sich bei Anwendung der Erfindung in Vergleichen mit Koronarogrammen von Koronarkranken bestätigt, und zwar so : in Linksseitenlage kommt es bei Ischämie im Gebiet der re.Koronararterie zum Abweichen des T-Vektors nach vorne (αT wird grösser als 110°). Damit kommt es auch zu einer Oeffnung bzw. Vergrösserung des Winkels Phi, welcher ja den wahren Winkel im Raum zwischen den Maximalvektoren QRS und T bezeichnet. Ein KGM in Linkslage als kleiner Funktionstest der RCA sollte deshalb routinemässig immer mitgeschrieben werden.

Serienmessungen bei Patienten mit Koronarinsuffizienz zeigen bei technisch einwandfreien Projektionen x, y, z ein mehr oder weniger starkes Schwanken der Werte für den T-Vektor. Die Standardabweichung von zehn Messungen liegt also über dem arbitrarisch festgelegten Wert 5°, und dies ist wahrscheinlich ein Hinweis für das Vorliegen einer Myokardischämie. Erstmals konnte dieses Phänomen des « floating » bei einem Patienten im status anginosus kurz von Auftreten eines Vorderwandinfarktes mit Hilfe des Kardiogoniometers und Ableitungsverfahren gemäss Erfindung beobachtet werden.

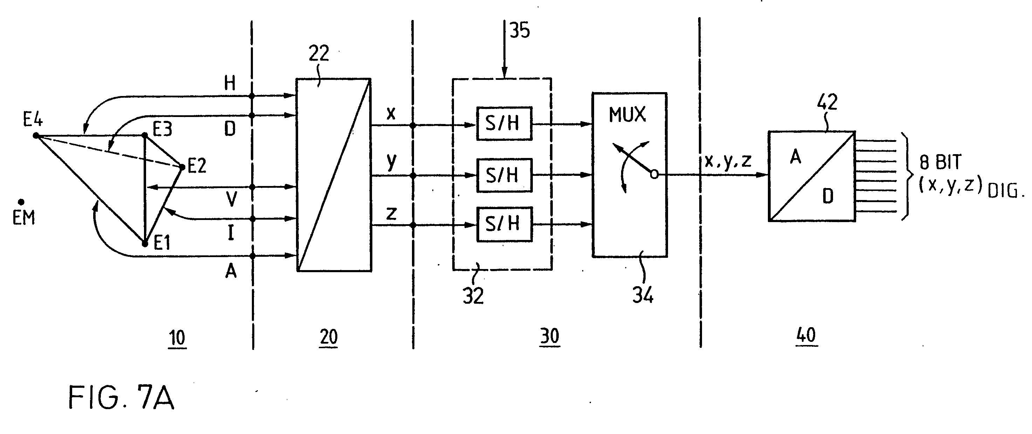

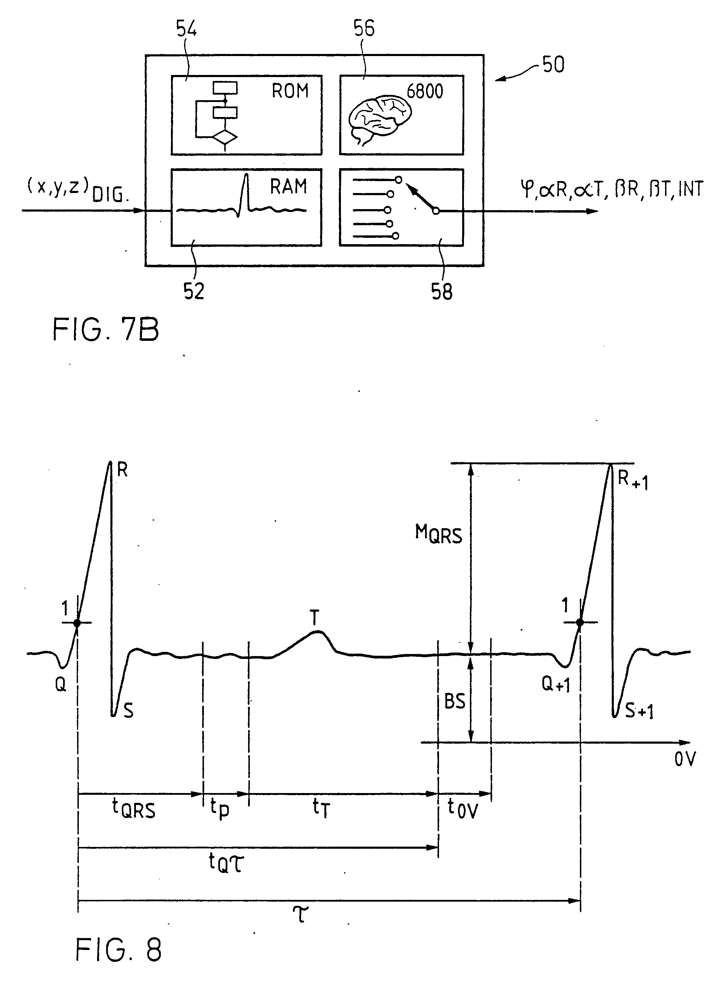

Die Figuren 7A und 7B zeigen in vereinfachter Darstellung die Vorrichtung zur Druchführung des Verfahrens. In Figur 7A ist die Informationsgewinnung ab dem meschlichen Körper bis zu Digitalisierung in vier apparative Stufen 10, 20, 30 und 40 aufgeteilt. Die erste mit 10 bezeichnete Stufe zeigt die Ableitungen, wie sie auch in Figur 1 dargestellt sind. Diese Ableitungen werden nach dem Ableitungsverfahren gemäss der Erfindung mit Hilfe von vier Thorax-Elektroden E1 bis E4 und einer Masse-Elektrode, die vorzugsweise am rechten Arm befestigt ist, gewonnen. Von den aus diesem Tetraeder prinzipiell gewinnbaren sechs Ableitungen sind deren drei linear unabhängig, und es werden in diesem Beispiel fünf verwendet ; die nicht näher bezeichnete Ableitung zwischen den Punkten E2 und E3 wird als zur nicht interessierenden Projektionsebene gehörend nicht benützt bzw. als redundante Paarung weggelassen. Die benützten fünf Ableitungen sind mit H für Horizontal, D für Dorsal, V für Vertikal, I für Inferior und A für Anterior bezeichnet. Die elektrischen Signale dieser fünf gewonnen Ableitungen werden von der Ableitungsstufe 10 in die Koordinatentransformationsstufe 20 geführt, in der die gesuchten Projektionen in ein orthogonales System vorgenommen werden. Dies wird durch ein vorzugsweise analoges Netzwerk 22 durchgeführt, wobei auch direkt drei linear unabhängige Ableitungen in einer digitalisierenden Koordinatentransformationsstufe 20 verarbeitet werden können. Die nun gewonnen Projektionen x, y, z gelangen von der Koordinatentransformationsstufe 20 in eine Abtaststufe 30, wo sie von einer Abtastschaltung 32 (Sample-and-Hold-Schaltung) abgetastet werden. Zur Abtaststufe 30 gehört auch eine Multiplexschaltung 34 zur Parallel-in-Serie-Umsetzung der gewonnen Projektionen.

Von der Abtaststufe 30 gelangen die nun seriell anfallenden Daten der Einzelprojektionen in eine Digitalisierstufe 40, die in vereinfachter Darstellung durch einen Analog-Digitalumsetzer 42 dargestellt ist. Die x-, u- und z-Projektionen werden in dieser Stufe beispielsweise mit 8-Bit-Auflösungen digitalisiert ; diese Auflösung hat sich für die üblichen praktischen Zwecke als genügend gut erwiesen, doch könnten zur verfeinerten Darstellung der Messungen bzw. zum Aufsuchen noch unbekannter Effekte innerhalb der elektrischen Signale auch eine 16-Bit- oder noch höhere Auflösung vorgesehen sein. Symbolisch sind die digitalisierten Projektionen mit $(x, y, z)_{dig}$ dargestellt.

Figur 7B zeigt nun in sehr vereinfachter Darstellung den Hauptteil 50 des Kardiogoniometers in welchem die digitalisierten Projektionen $(X, y, z)_{dig}$ in die entsprechenden aussagekräftigen Grössen umgesetzt werden. Gemäss dem vorher beschriebenen Beispiel sind dies der Winkel Phi, das ist der wahre Winkel im Raum zwischen den QRS- und T-Maximalvektoren, dann die auf das zugrundegelegte Koordinatensystem, das im Verfahren gemäss der Erfindung kein orthogonales ist — die Ableitungen sind nicht orthogonal, wohl aber die als Daten verwerteten, aus den Ableitungen errechneten Projektionen x, y, z —, bezogenen Werte $\alpha R$, $\alpha T$, $\beta R$ und $\beta T$ sowie das beispielsweise zwischen je zwei QRS-Flanken abgeleitete Schlaginterval des Herzens. Der Kardiogoniometerteil 50 ist dargestellt durch einen, die aktuellen Herzschlagdaten festhaltenden Speicher 52 mit wahlfreiem Zugriff, einen das Verarbeitungsprogramm enthaltenden Speicher 54, der für das Datenmanagement notwendige Mikroprozessor 56, der beispielsweise vom Motorola-Typ 6800 sein kann, wie er dies im beschriebenen Kardiogoniometer auch ist, sowie in symbolischer Darstellung ein Schaltnetzwerk 58, z. B. ein Bussystem zur Bereitstellung der interessierenden Daten aus einem nicht dargestellten Zwischenspeicher, welche natürlich auch seriell aus dem Kardiogoniometer herausgeführt werden können. Streng genommen fallen diese Daten erst nach dem Umsetzen in peripheren Geräten wie beispielsweise in einer Anzeigeeinheit, einem Drucker, einem Bildschirm an, doch soll diese sehr vereinfachte Darstellung eine überhaupt nicht ins Detail gehende Uebersicht bringen, wie es von den am Patienten angebrachten Elektroden durch Signal- sowie Datenverarbeitung zu den diagnostisch sehr aussagefähigen Resultaten kommt.

Figur 8 dient zur Veranschaulichung der zeitabhängigen Verfahrensschritte eines Herzschlagintervalls. Das typische Bild einer EKG-Kurve ist ausschnittsweise dargestellt, und zwar in der Zeitdauer von einem vollerfassten QRS-Komplex zu einem teilerfassten $QRS_{+1}$-Komplex. Die steile QR-Flanke eigent sich erfahrungsgemäss besonders gut zur Triggerung von Funktionsabläufen. Mit der QR-Triggerung an einer Schwelle 1 beginnt die Messung des Schlagintervalls $\tau$ sowie die Aufteilung von $\tau = (QRS$ bis $QRS_{+1})_t$ in diverse Zeitfenster $t_{QRS}$; $t_p$; $t_T$; $t_{OV}$; $t_{QT}$, d. h. in einzeln abzuspeichernde Intervalle, wobei die Zeitdauer $t_{OV}$ eine zentrale Rolle spielt; an dieser Stelle wird die Basislinie des Kurvenzuges gemessen, dann gemittelt und mit dem elektrischen Neutralpunkt Null-Volt (OV) verglichen. Diese Basislinienspannung BS dient als Korrekturwert für sämtliche amplitudenabhängigen Grössen wie beispielsweise die eingezeichnete QRS-Magnitude $M_{QRS}$; sie stellt eigentlich die biologische Null-Linie dar.

Figur 9 zeigt nunmehr detailliert die Kardiogoniometereinrichtung. Die elektrischen Messwerte der fünf Ableitungen D, A, I, V, H, gelangen über einzelne Eingangsverstärker 24 auf ein Analogrechennetzwerk 25, das im wesentlichen die Schaltung zur Koordinatentransformation 22 darstellt, in welchem Rechennetzwerk die Projektionen x, y, z gemäss der angegebenen Formeln ermittelt und die Signale der Projektionsgrössen bereitgestellt werden. Diese Signale werden anschliessend in Ausgangsverstärkern 26 deswegen verstärkt, dass sie in einem parallel laufenden Kardiographen bzw. dessen Schreiber mitgeschrieben werden können. Dafür ist eine Sammelleitung 29 vorgesehen.

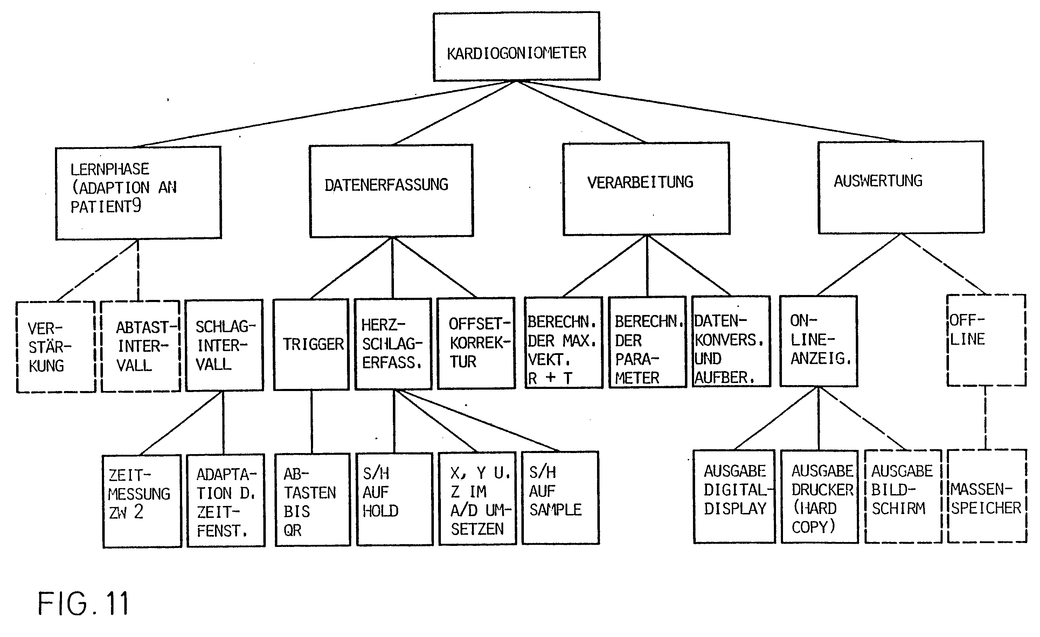

Die x-, y-, z-Signale werden über jeweilige Verstärker 31 den Sample-and-Hold-Schaltungen 32 zugeführt, deren Sample-Zeiten über die Leitung 35 von einem Steuerungsprogramm via die Steuerungsschaltung 53 bestimmt werden. Synchron dazu muss der Multiplexer 34 zum Abtasten der anstehenden Signale gesteuert werden, was über die Leitung 36 durch das gleiche Steuerungsnetzwerk 53 geschieht. Dieser beschriebene Schaltungsteil umfasst die Analogsignal-Verarbeitung, wobei die anfallenden Analogsignale im Analog-/Digitalwandler 42 zu digitalen Daten umgesetzt werden. In der angegebenen Ausführungsform wurde, wie schon erwähnt, eine 8-Bit-Auflösung gewählt, die jedoch ohne grossen Zusatzaufwand auf 16-Bit erweitert werden kann. Die digitale Datenverarbeitung umfasst dann eine Eingangsschaltung 51, einen Datenspeicher 52 und einen Steuerprogrammspeicher 54 sowie einen dazugehörigen Mikroprozessor 56. Für die Datenspeicherung wird ein Speicher mit wahlfreiem Zugriff verwendet, für die Speicherung des Steuerprogramms verwendet man vorzugsweise einen Speicher vom EPROM-Typ. Die gespeicherten Daten beziehen sich, wie in Figur 7B dargestellt, auf die digitalisierten Herzrhythmuskurven gemäss Figur 8, während das Steuerprogramm in prinzipieller Darstellung im Struktogramm des Verfarhens in Figur 11 dargestellt ist.

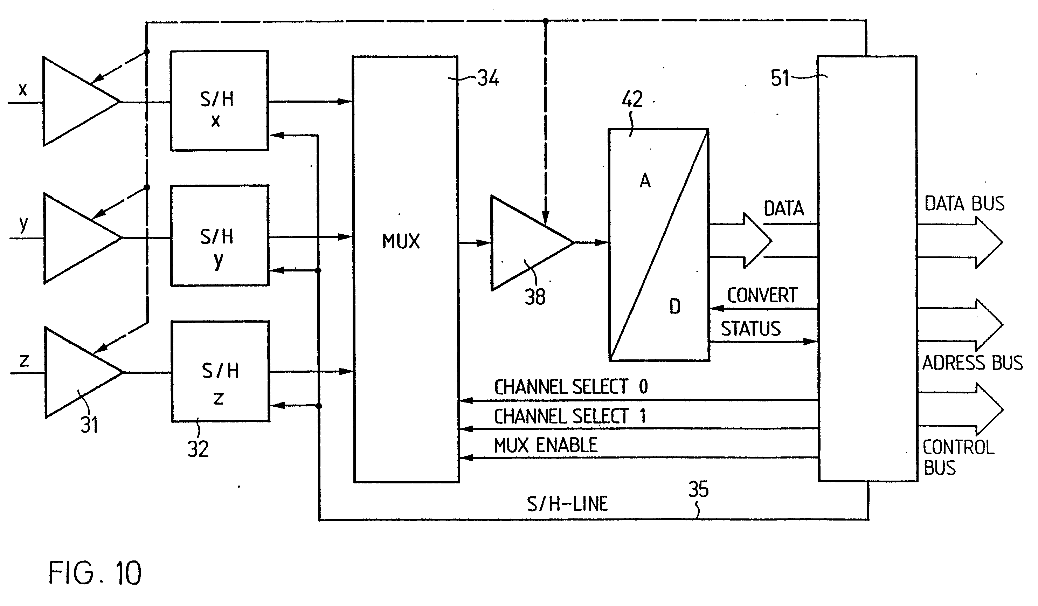

Vor der Beschreibung des Struktogramms soll noch auf eine detailliertere Darstellung des Analog/ Digitaladaptermoduls verwiesen werden. Dieses Netzwerk ist in Figur 10 dargestellt und umfasst die Bausteine der x-, y-, z-Eingangsverstärker 31, deren Ausgänge mit den jeweiligen Sample-and-Hold-Schaltungen 32 verbunden sind, die wiederum von der Eingangsschaltung 51 zum Mikrocomputer über das hier nicht dargestellte Steuerungsnetzwerk 53 und die Zuleitung 35 synchronisiert sind. Der Multiplexer empfängt neben den drei x-, y-, z-Datensignalen, hier noch in analoger Form, Kanalwählbefehle 0,1 sowie einen Multiplexer-Auslösebefehl 0,1 durch die digitale Eingangsschaltung 51, ebenso werden die Eingangsverstärker 31 sowie eine Zwischenverstärker 38 aus der gleichen Schaltung bezüglich der Verstärkung angesteuert. Die zu digitalisierenden Herzrhythmussignale aus dem Multiplexer 34 werden, wie schon erwähnt, im Verstärker 38 zwischenverstärkt und auf den Analog-/Digitalwandler 42 gebracht. Der Analog-/Digitalwandler steht mit der Eingangsschaltung 51 nebst dem Datenbus zusätzlich noch über eine Leitung für die Statusangabe in Verbindung; die Eingangsschaltung 51 startet den Analog-/Digitalwandler mittels eines Convertsignals über eine andere Leitung. Die Eingangsschaltung 51 ist mit dem Mikrocomputer wie üblich über drei Bussysteme verbunden, dem Datenbus, dem Adressenbus und dem Kontrollbus.

Figur 11 zeigt schliesslich ein Jackson-Struktogramm des Verfahrens in starker Vereinfachung. Struktogramme nach M. Jackson werden von oben nach unten und von links nach rechts gelesen. Folgt man diesem Struktogramm, so sieht man links in der Figur in einem ersten Schritt eine Lernphase, das ist die Adaptation des Gerätes an den Patienten, woraus dann Parameter für die Verstärkung, Abtastintervalle und Schlagintervalle resultieren. Dazu werden drei bis sechs Zyklen gemäss Figur 8 aufgewendet.

Aus dem Schlagintervall erhält man die Zeitmessung zwischen zwei Herzschlägen sowie die jeweils patientenspezifische Adaptation der verschiedenen, in Figur 8 dargestellten Zeitfenster. Anschliessend erfolgt eine Datenerfassung, aus welcher die Triggerpunkte, das Erfassen des Herzschlages, und die Offsetkorrektur primär resultieren. Die Herzschlagsignale werden kontinuierlich abgetastet, beispielsweise in 3ms Abständen. Die Messwerte einer Herzschlagperiode werden erst nach Detektion des Triggerpunktes abgespeichert und ausgewertet. Dieser Triggerpunkt wird bestimmt durch das Ueberschreiten einer vorgegebenen Steilheit der QR-Flanke. Triggerpunktsuche und Herzschlagerfassen werden nun und später ständig wiederholt, was in Form eines kleinen Sterns oben rechts im Funktionsblock angegeben ist. Bei der Datenumsetzungsgeschwindigkeit der besprochenen Ausführungsform lässt sich leicht jeder dritte Herzschlag erfassen. Mit dem nötigen Zusatzaufwand kann auch jeder Herzschlag erfasst werden. Die Verarbeitung der Signale bezieht sich in der vorläufigen Entwicklungsstufe auf das Berechnen der Maximalvektoren R und T, Berechnen der Grössen Phi, $\alpha R$, $\alpha T$, $\beta R$, $\beta T$, usw. sowie auf die Datenkonversion und -aufbereitung. Schliesslich gelangen die errechneten grössen zur Beurteilung in sogenannte Online-Anzeigen, d. h. Messwertanzeigen zur Diagnose während der Messung am Patienten, gerne Bedside-Methode genannt. Darunter fallen die Messdatenausgabe auf einer Digitalanzeige, die Ausgabe derselben und weiterer Daten auf einen Drucker zur Registrierung und für Archivzwecke sowie die Ausgabe auf einen Bildschirm zur Verfolgung einer grafischen Darstellung. Dann werden in sogenannten Offline-Auswertungen, also weg vom Patienten, gespeicherte Signale aus dem Speicher 52 für weitere Datenverarbeitung bereitgestellt beispielsweise Statistiken, usw., die auf Massenspeicher übertragen mit entsprechend aufwendigen Programmen in grösseren Computern weiter ausgewertet werden können.

Mit Hilfe der Elektrokardiographie gemäss Stand der Technik ist es, da sie nur Messungen in einer Ebene tätigt, nicht möglich, die elektrischen Vorgänge des Herzens am gleichen Herzschlag ganz zu erfassen. Dies ist nur mit einem dreidimensionalen Ableitungssystem möglich, eben der Vektorkardiographie. Die Kardiogoniometrie als erste Stufe einer Bedside-Vektorkardiographie verwendet nun ein neues, technisch so einfaches, dreidimensionales Ableitungssystem, dass damit die dreidimensionale Erfassung der elektrischen Vorgänge am Krankenbett in Serie möglich wird. Sie erfasst vorerst einzig die Maximalvektoren der QRS-Schlinge und der T-Schlinge. Der Informationsgehalt dieser beiden Messgrössen ist aber schon sehr gross.

Bis heute war es auch nicht möglich, komplexere Auswertungen von gemessenen Werten gleich am Krankenbett zu erhalten. Ueblich ist, wie dies beispielsweise in der US-PS-4.106.495 von Harold Kennedy 1977 vorgeschlagen ist, Messdaten am Körper zu sammeln und aufzuzeichnen, um sie anschliessend mit mehr oder weniger Zeitabstand auf einem Rechensystem auszuwerten. Nicht selten war ein Patient schon tot, bevor seine Daten zur Auswertung an die Reihe kamen. Die praktisch unmittelbare Erfassung, Verwertung und Darstellung der komplexen Zusammenhänge innerhalb eines oder nur weniger Herzschläge entspricht einem Bedürfnis. Die Online-Auswertung ermöglicht sozusagen in Echtzeit, bei Medikamentenanwendung auf dem Ergometer und bei Manipulationen aller Art die Reaktion des Herzens zu beobachten, beispielsweise auf dem Bildschirm.

Der QRS-Vektor entspricht der sogenannten elektrischen Herzachse. Ihre Lage im Raum wird heute qualitativ mit den Begriffen Querlage, Steillage, Horizontallage, überdrehter Linkstyp, usw. charakterisiert. Statt dieser qualitativen Bezeichnungen liefert die Kardiogoniometrie (KGM) eine eindeutig im Raum definierte Richtung. Schon kleine Veränderungen dieser Richtung lassen sich somit im Laufe der Zeit nachweisen, z. B. zu- oder Abnahmen von Herzdilatationen, Zu- oder Abnahmen von Herzhypertrophien, Entwicklung eines linksanterioren, faszikulären Blockes, usw. ; dies alles, bevor im Standard-Elektrokardiogramm, also bei Messungen gemäss dem Stand der Technik, irgendeine wesentliche Aenderung zu bemerken ist. Veränderungen der QRS-Vektors im Akutversuch, wie beispielsweise Lagewechsel des QRS-Vektors in Linksseitenlage oder nach Belastung oder nach Verabreichung von Medikamenten wie z. B. Nitroglyzerin, lassen lokale, kleine Zirkulationsstörungen in den Septalästen der Koronararterien nachweisen.

Lageveränderungen des T-Vektors erlauben einen Einblick in die Verhältnisse der Repolarisation des Herzens, d. h. geben Hinweise auf Stoffwechselstörungen oder Durchblutungsstörungen im Myokard. Besteht eine Ischämie der Hinterwand (RCA), findet sich der R-Vektor nicht mehr im Normbezirk, sondern weiter vorne, umgekehrt aber bei einer Ischämie der Vorderwand (LCA) findet er sich ausserhalb der Normgrenze mehr hinten. Natürlich zeigen sich diese Veränderungen auch bei lokalisierten Stoffwechselstörungen der Hinter- bzw. Vorderwand. Ist das Myokard in Ruhe noch genügend durchblutet, so ist der T-Vektor möglicherweise noch nicht aus der Normgrenze abgewichen. Hier kann die relative Ischämie, also eine latente Koronarinsuffizienz manifest gemacht werden, durch Verminderung des Koronardurchflusses, z. B. nach Gabe von Nitroglyzerin — Nitroglyzerin führt einerseits zu einer Senkung des preload und des Schlagvolumens, damit auch zu einer Blutdrucksenkung, andererseits zu einer Herzschlagfrequenzzunahme. Beide Effekte führen zu einer Verminderung des Koronardurchflusses,

EP 0 086 429 B1

welcher sich besonders in bereits leicht stenosierten Koronarien auswirkt. Damit kommt es zu einem Abwandern des T-Vektors von der ischämischen Myokardstelle weg. Dieser Effekt ist so eindeutig, dass bei Koronarkranken auf die Ergometrie verzichtet werden kann.

Ist eine Koronarstenose nicht vollständig (Verschluss), kann es zu einem Wandern des T-Vektors in einem mehr oder weniger begrenzten Gebiet kommen. Diesen schon vorher erwähnten und mit Floating bezeichneten Effekt findet man die kardiogoniometrischen Serienmessungen gemäss Erfindung. Dieser Effekt ist äusserst sensibel und kann, z. B. auch nach kurzen Herzstillständen, z. B. nach karotis-Sinusdruck, demonstriert werden.

Da diese beiden Vektoren sehr sensibel auf jegliche Zirkulationsstörungen im Herzen reagieren, dürfte die Kardiogoniometrie auch zur Ueberwachung von Herzpatienten in den Ueberwachungsstationen und bei Patienten nach Myokardinfrakten oder Herzoperationen gute Dienste leisten.

Die Kardiogoniometrie als nichtinvasive, völlig nebenwirkungsfreie Üntersuchungsmethode eignet sich ebenfalls zur regelmässigen, alljährlichen Herzkontrolle zur frühzeitigen Erfassung latenter Koronarinsuffizienzen bei Myokardinfarkt-gefährdeten Patienten, wie es beispielsweise Raucher, Diabetiker, Manager und Hypercholesterinämiker sind. Da die Methode sehr empfindlich auf Durchblutungsstörungen der Koronararterien unmittelbar reagiert, dürfte sie sich auch zur Ueberprüfung von herzaktiven Medikamenten eignen.

Da im Kardiogoniometer nicht bloss die Maximalvektoren, sondern beide Vektorschlingen in toto gespeichert sind, können, wie schon erwähnt, in Offline-Verfahren weitere Grössen gewonnen werden ; beispielsweise beliebige Zeitintervalle wie $tQR_{max}$ und QR oder $tQT_{max}$ und QT, die Darstellung eines Vektors X zur zeit $^tX$, beispielsweise Initialvektoren ; dann ist auch die Visionalisierung auf einem Bildschirm, und zwar nicht bloss der Maximalvektoren, sondern auch der ganzen Schleife von QRS und T, einzeln oder in Serie zur Sichtbarmachung, beispielsweise eines allfälligen Floatings, möglich. Es ist der eigentliche Weg zu einer wirklich genauen und völlig computerisierten Herzdiagnostik, wobei die Ausführungsform des vorgestellten, beschriebenen Kardiogoniometers der handlichen Grösse wegen ausserordentlich praktisch im Ambulanten Betrieb eingesetzt werden kann.

**Patentansprüche**

1. Verfahren zur Ermittlung und Darstellung eines auf das Herz bezogenen Raumvektors, der den Vektor des elektrischen Feldes darstellt, das durch die Aktivitaet des Herzens gebildet wird, ausgehend von Messwerten, die an Ableitungspunkten am Koerper erfasst werden, wobei aus den Messwerten an je zwei Ableitungspunkten je eine Potentialdifferenz gebildet wird, dadurch gekennzeichnet, dass ein erster Ableitungspunkt (E1) an einer Stelle vorgesehen wird, wir sie nach bekannter Weise zur Ermittlung einer Spannung V4 nach Wilson verwendet wird, dass ein zweiter Ableitungspunkt (E2) an einer Stelle vorgesehen wird, wie sie nach bekannter Weise zur Ermittlung einer Spannung V8 nach Wilson verwendet wird, dass ein dritter Ableitungspunkt (E3), im wesentlichen lotrecht zur Verbindungslinie des ersten mit dem zweiten Ableitungspunkt, ueber dem ersten Ableitungspunkt (E1) in einem Abstand von Faktor 0.6 bis 0.8, multipliziert mit der Distanz zwischen dem ersten und dem zweiten Ableitungspunkt, vorgesehen wird, dass ein vierter Ableitungspunkt (E4), imwesentlichen waagrecht und unter Bildung eines ungefaehren rechten Winkels zur Verbindungslinie des ersten (E1) und dritten (E3) Ableitungspunktes, und ausgehend vom dritten Ableitungspunkt (E3) in Richtung zur rechten Koerperseite, im Abstand von Faktor 0.6 bis 0.8, multipliziert mit der Distanz zwischen dem ersten und dem zweiten Ableitungspunkt (E1, E2) vorgesehen wird, so dass Potentialdifferenzen gemessen werden koennen, zwischen dem ersten und zeiten (I), ersten und vierten (A), zweiten und vierten (D), dritten und vierten (H) und ersten und driten (V) Ableitungspunkt, dass die gemessenen Potentialdifferenzen direkt als Teilvektoren verwendet werden und dass daraus der Raumvektor (V) durch vektorielle Addition ermittelt wird.

2. Verfahren gemaess Anspruch 1, dadurch gekennzeichnet, dass zur Ermittlung des Raumvektors solche Potentialdifferenzen verwendet werden, die zwischen Ableitungspunkten (E1, E3, E4 und E1, E2, E4) auftreten, die zusammen in zwei Ebenen liegen.

3. Verfahren gemaess Anspruch 1, dadurch gekennzeichnet, dass aus dem Raumvektor (V) orthogonale Projektionen (x, y, z) berechnet werden.

4. Verfahren gemaess Anspruch 1, dadurch gekennzeichnet, dass die Messwerte, die an den Ableitungspunkten (E1, E2, E3, E4) gemessen werden, gespeichert werden.

5. Verfahren gemaess Anspruch 3, dadurch gekennzeichnet, dass die orthogonalen Projektionen (x, y, z) zur Darstellung von Vektoren und ihren Raumwinkeln und durch die Vektoren gebildeten Schleifen verwendet werden.

6. Verfahren gemaess Anspruch 3 oder 5, dadurch gekennzeichnet, dass die Werte der orthogonalen Projektionen und oder der daraus errechneten Zwischen- und/oder Endwerte von mehreren Herzschlaegen einer Messung, in Speichern gespeichert und mit statistischen Berechnungen verarbeitet und daraus abgeleitete auf das Herz bezogene Werte aufgezeichnet werden.

**Claims**

1. Method for detecting and representing a heart-related spatial vector which represents the vector

EP 0 086 429 B1

of an electric field produced by the activity of the heart, starting from measured values taken at lead points on the body, whereas values measured at each pair of lead points are used to determine potential differences, characterized in that a first lead point (E1) is positioned at a point as used for determining a tension V4 in a well known way according to Wilson, in that a second lead point (E2) is positioned at a point as used for determining a tension V8 in a well known way according to Wilson, in that a third lead point (E3) is positioned generally vertically upwardly with respect to a line connecting the first and second lead points and above the first lead point (E1) at a distance equal to the distance between the first and second lead points multiplied by a factor having a value between 0.6 and 0.8, in that a fourth lead point (E4) is positioned along a line generally horizontal and perpendicular to the line between the first (E1) and third (E3) lead points and toward the right body side therefrom at a distance equal to the distance between the first and second lead points (E1, E2) multiplied by a factor having a value between about 0.6 and 0.8 such that potential differences can be computed between the first and the second (I), the first and the fourth (A), the second and the fourth (D), the third and the fourth (H) and the first and the third (V) lead points and such that the measured potential differences are directly taken as partial vectors and that said spatial vector (V) is established by vectorial addition.

2. Method according to claim 1, characterized in that potential differences that occur between lead points (E1, E2, E3, E4) being in two planes are used for determining the spatial vector.

3. Method according to claim 1, characterized in that orthogonal projections (x, y, z) are computed from the spatial vector (V).

4. Method according to claim 1, characterized in that values measured in lead points (E1, E2, E3, E4) are stored.

5. Method according to claim 3, characterized in that orthogonal projections (x, y, z) are used for representing vectors and their angles in space and for representing slopes formed by these vectors.

6. Method according to claim 3 or 5, characterized in that values of the orthogonal projections and/or of intermediate values and/or of end values calculated therefrom for a plurality of heartbeats are stored in memories and processed in statistical calculations and in that heart-related values derived therefrom are recorded.


## Revendications

1. Procédé pour déterminer et représenter dans un espace un vecteur qui se réfère au cœur et qui représente le vecteur d'un champ électrique produit par l'activité du cœur, partant de valeurs mesurées qui sont prises à différents points de dérivation au corps et où une différence de potentiel est chaque fois dérivée des valeurs mesurées en deux points de dérivation, caractérisé en ce que un premier point de dérivation (E1) est prévu à une place correspondant à celle utilisée pour déterminer une tension V4 de manière connue d'après Wilson, en ce que un deuxième point de dérivation (E2) est prévu à une place correspondant à celle utilisée pour déterminer une tension V8 de manière connue d'après Wilson, en ce que un troisième point de dérivation (E3) est prévu à une distance du premier point (E1) qui correspond à celle qui sépare le premier et le second point multipliée par un facteur entre 0.6 et 0.8 et situé généralement verticalement au dessus du premier point par rapport à une ligne qui relie le premier et le second point, en ce que un quatrième point de dérivation (E4) est prévu le long d'une ligne généralement horizontale qui forme un angle à peu près droit avec une ligne qui relie le premier (E1) et le troisième point (E3) de dérivation et à une distance partant du troisième point (E3) vers la droite du corps qui correspond à la distance qui sépare le premier (E1) et le second (E2) point de dérivation multipliée par un facteur entre 0.6 et 0.8, afin que des différences de potentiel puissent être mesurées entre le premier et le second (I), le premier et le quatrième (A), le deuxième et le quatrième (D), le troisième et le quatrième (H) et le premier et le troisième (V) point de dérivation et en ce que les différences de potentiel mesurées sont directement utilisées comme vecteurs partiels et que le vecteur (V) dans un espace est calculé par une addition vectorielle.

2. Procédé selon la revendication 1, caractérisé en ce que les différences de potentiel utilisées pour déterminer le vecteur dans l'espace sont celles qui existent entre les points de dérivation (E1, E3, E4 et E1, E2, E4) qui forment ensemble deux plans.

3. Procédé selon la revendication 1, caractérisé en ce que des projections orthogonales (x, y, z) sont calculées à partir du vecteur (V) dans l'espace.

4. Procédé selon la revendication 1, caractérisé en ce que les valeurs qui sont mesurées aux points de dérivation (E1, E2, E3, E4) sont mémorisées.

5. Procédé selon la revendication 3, caractérisé en ce que les projections orthogonales (x, y, z) sont utilisées à représenter les vecteurs et leurs angles dans l'espace ainsi que les boucles formées par ces vecteurs.

6. Procédé selon la revendication 3 ou 5, caractérisé en ce que les valeurs des projections orthogonales ou/et les valeurs intermédiaires ou/et les valeurs terminales de plusieurs battements du cœur sont mémorisées dans des mémoires, traitées dans des calculs statistiques et que des valeurs ainsi obtenues sont enregistrées.

11

EP 0 086 429 B1

FIG. 1

FIG. 3

FIG. 2

FIG. 4

1

| HI | RA | RB | TA | TB | INT |
|---|---|---|---|---|---|
| 15 | 104 | 12 | 120 | 14 | 77 |
| 12 | 104 | 9 | 116 | 15 | 82 |
| 14 | 103 | 8 | 117 | 13 | 82 |
| 8 | 103 | 10 | 112 | 13 | 83 |
| 9 | 105 | 9 | 113 | 13 | 86 |
| 10 | 105 | 10 | 115 | 13 | 85 |
| 10 | 104 | 11 | 115 | 14 | 84 |
| 7 | 106 | 10 | 113 | 14 | 87 |
| 10 | 102 | 7 | 112 | 12 | 89 |
| 8 | 106 | 9 | 114 | 10 | 84 |

FIG. 5

FIG. 6

FIG. 6'

FIG. 7A

EP 0 086 429 B1

FIG. 7B

FIG. 8

FIG. 9

FIG. 10

KARDIOGONIOMETER

LERNPHASE (ADAPTION AN PATIENT9

VER-STÄR-KUNG

ABTAST-INTER-VALL

SCHLAG-INTER-VALL

ZEIT-MESSUNG ZW 2

ADAPTA-TION D. ZEIT-FENST.

DATENERFASSUNG

TRIGGER

HERZ-SCHLAG-ERFASS.

OFFSET-KORREK-TUR

AB-TASTEN BIS QR

S/H AUF HOLD

X, Y U. Z IM A/D UM-SETZEN

S/H AUF SAMPLE

VERARBEITUNG

BERECHN. DER MAX. VEKT. R + T

BERECHN. DER PARA-METER

DATEN-KONVERS. UND AUFBER.

AUSWERTUNG

ON-LINE-ANZEIG.

OFF-LINE

AUSGABE DIGITAL-DISPLAY

AUSGABE DRUCKER (HARD COPY)

AUSGABE BILD-SCHIRM

MASSEN-SPEICHER

FIG. 11

EP 0 086 429 B1